# EUROPÄISCHE PATENTSCHRIFT

(11) **EP 1 779 822 B1**
(45) Veröffentlichungstag und Bekanntmachung des Hinweises auf die Patenterteilung: **16.01.2008**
(21) Anmeldenummer: 05025858.1
(22) Anmeldetag: 26.11.2005
(51) Int. Cl.: A61F 5/41

(54) **Zugvakuumvorrichtung zur Penisextension**
Device for extending a penis by applying vacuum generated by pulling
Dispositif d'allongement du pénis en créant le vide par traction

(30) Priorität: 26.10.2005 DE 102005051528
(43) Veröffentlichungstag der Anmeldung: 02.05.2007
(73) Patentinhaber: Höfer, Jürgen, 20146 Hamburg (DE)
(72) Erfinder: Höfer, Jürgen, 20146 Hamburg (DE)

(56) Entgegenhaltungen:
- WO-A-95/10991
- US-A- 5 836 864
- US-B1- 6 458 073

## Beschreibung

Die vorliegende Erfindung betrifft eine Zugvakuumvorrichtung zur Penisextension, umfassend einen Hohlkörper (2), welcher die problemlose Einführung eines Penis (1) ermöglicht, wobei der eingeführte Penis (1) durch eine diesen und den Hohlkörper (2) dichtend verbindenden Hülle (14) (Standard-Kondom (5) oder Penis-Manschette (6)) luftdicht umhüllt ist. Bei Einleitung einer Zugkraft auf eine am hermetisch abgeschlossenen Hohlkörper (2) befestigten Zugvorrichtung (7) wird im Hohlkörper (2) ein Unterdruck erzeugt, wodurch mittels der hierdurch erzeugten Saugkraft im Hohlkörperinnenraum (3) bei kontinuierlicher Anwendung eine Dehnung des in den Hohlkörper (2) eingeführten Penis (1) hervorgerufen wird.

### Stand der Technik

Bekannt sind diverse Vorrichtungen zur Penisextension, wobei die Wirkung der folgenden Vorrichtungen auf der Erzeugung eines Vakuums basiert: In US 4,753,227, US 5,306,227, US 5,836,864 und US 6,926,666 wird für die Erzeugung des Vakuums eine Pumpe benötigt. Schließlich befindet sich eine Vorrichtung der Firma Phalomed Manufacturing Ltd., Malta, unter der Markenbezeichnung "Phallosan" auf dem Markt, bei welcher ein Vakuum durch Zug an einem Spezialstreckkondom ausgeübt wird. Das Prinzip des "Phallosan" wird in DE 10001331A1 vorgestellt.

### Kritik des Standes der Technik

Die vorliegende Erfindung steht im Gegensatz zu bekannten Penisextensionsgeräten, bei denen ein Vakuum zur Penisdehnung Verwendung findet, nämlich US 4,753,227, US 5,306,227, US 5,836,864 und US 6,926,666, da bei diesen Vorrichtungen Pumpen zur Erzeugung eines Vakuums benötigt werden, was diese Vorrichtungen unhandlich und umständlich in der Bedienung macht. Außerdem können diese - anders als die vorliegende Erfindung - nicht diskret unter der Kleidung getragen werden, was eine Anwendung lediglich in der privaten Sphäre des Anwenders ermöglicht. Dies resultiert wiederum in einer relativ geringen täglichen Anwendungsdauer, die sich in einem für die betroffenen Männer unbefriedigenden Erfolg niederschlägt.
Die vorliegende Erfindung steht ebenfalls im Gegensatz zu einem in der Offenlegungsschrift DE 10001331A1 vorgeschlagenen der Eichel eng anliegenden Kondom-,Hütchen'. Unterstützt durch den unmittelbar hinter der Eichel dichtend an dem Penisschaft anliegenden Ringabschnitt des Kondoms bildet sich unter dem 'Hütchen' bei Einleitung der durch ein Dehnelement ausgeübten Dehnungskraft ein Unterdruck aus. Leicht abgeändert ist die vorerwähnte Vorrichtung unter der Markenbezeichnung "Phallosan" auf dem Markt. Der "Phallosan" weist eine Reihe von gravierenden Nachteilen auf, wovon hier nur die wichtigsten Erwähnung finden können:
**Beim "Phallosan"**
   1. muss das verwendete Dauerstretch-Spezialkondom sowohl proximal als auch distal vollkommen luftdicht abgeschlossen werden, um funktionsfähig zu sein.
   2. wird ein sehr teures extrem dickwandiges Dauerstretch-Spezialkondom verwendet, dessen Form einem Urinalkondom ähnelt. Es endet an der Spitze in einem Schlauch, der deswegen extrem dickwandig ausgebildet ist, um an ihm Zug ausüben zu können.
   3. muss das Ende des Schlauchstücks exakt mittig in einen Spezialclip gepresst werden, was Geschicklichkeit und eine erhebliche Kraftaufwendung erfordert.
   4. entsteht die Sogwirkung nur an Eichel und Vorhaut
   5. müssen Eichel und Vorhaut in die glockenförmige Erweiterung des Dauerstretch-Spezialkondoms gezwängt werden, was ein nicht unproblematisches und etwas unhygienisches Procedere erfordert.
   6. wird das Spezialkondom beim Wasser lassen unvermeidlich benetzt.
   7. erfolgt leicht ein Ankleben des sehr dickwandigen Dauerstretch-Spezialkondoms beim Zurückrollen, was die Verwendung desselben zunächst erschwert und später unmöglich macht.
**Bei der vorliegenden Erfindung**
   1. besteht das Problem des distalen luftdichten Abschlusses überhaupt nicht, da der Hohlkörper (2) entweder distal überhaupt keine Öffnung hat oder eine Öffnung mit einem absolut luftdichten Verschluss (10) aufweist.
   2. kann ein normales Standardkondom oder eine handelsübliche Penis-Manschette verwendet werden, und die Zugvorrichtung ist nicht am Kondom, sondern am Hohlkörper (2) befestigt.
   3. wird kein Spezialclip, sondern nur ein simples Element, wie z.B. ein (Karabiner-) Haken benötigt.
   4. ist neben Eichel und Vorhaut auch ein Teil des Penis-Schaftes vom Vakuum umgeben, so dass auch der Penisschaft der Sogwirkung ausgesetzt ist.
   5. erfolgt die Einführung von Eichel, Vorhaut und Penis-Schaft in den Hohlkörper (2) absolut problemlos und ohne die Erfordernis des Hineinzwängens.
   6. kann durch die Öffnung des Verschlusses (10) uriniert werden, ohne dass der Hohlkörper (2) oder die Hülle (14) benetzt werden.
   7. können normale handelsübliche Standard-Kondome (5) oder alternativ Penis-Manschetten (6) verwendet werden, bei denen das Problem des Festklebens nicht auftritt.

### Aufgabe

Aus den vorstehend erläuterten gravierenden Nachteilen gattungsgemäßer Penisextensionsgeräte leitet sich die der vorliegenden Erfindung zugrundeliegende Aufgabenstellung ab, die darin besteht, ein Penisextensionsgerät mit den eingangs erläuterten Vorteilen zu schaffen, das unauffällig am Körper getragen werden kann. Die Erfindung hat die Aufgabe, den Leidensdruck durch subjektiv empfundene oder tatsächlich vorhandene Penishypoplasie durch eine vollkommen schmerzlose, unkomplizierte und kostengünstige Penisextension (Verdickung und Verlängerung des Penis) zu beseitigen. Der Erfindung liegt außerdem die Aufgabe zugrunde, eine Vorrichtung zu schaffen, die a) Patienten mit Prostatakrebs nach einer postoperativen Penisretraktion durch den Hormonentzug unkompliziert und kostengünstig helfen kann und die b) insbesondere auch für die Anwendung bei Querschnittsgelähmten geeignet ist, bei denen das Phänomen des "Retractio Penis" (Zurückziehung des Gliedes in das Unterhautfettgewebe) wegen der Lähmung der Bauchmuskulatur und die sitzende Position im Rollstuhl verstärkt auftritt. Hier eröffnet sich die Möglichkeit, das durch die Retraktion entstandene Mikroglied durch vollkommen schmerzlose kontinuierliche Dehnung auf Basis der hier vorgestellten Traktionsvakuum-Sogtechnik wieder aus dem Fettgewebe zu ziehen und gleichzeitig eine objektive Verlängerung und Verdickung des gesamten Peniskörpers (nicht nur der Eichel) zu erzielen.

### Lösung

Gemäß der vorliegenden Erfindung wird diese Aufgabe durch eine Zugvakuumvorrichtung gelöst, welche dem Anwender ermöglicht, bei Einleitung einer Zugkraft auf eine an einem hermetisch abgeschlossenen Hohlkörper (2) befestigte Zugvorrichtung (7) im Hohlkörper (2) einen Unterdruck zu erzeugen, wodurch mittels der hierdurch erzeugten Saugkraft im Hohlkörperinnenraum (3) bei kontinuierlicher Anwendung eine Dehnung des in den Hohlkörper (2) eingeführten Penis (1) hervorgerufen werden kann, und zwar durch die kontinuierliche Dehnungswirkung einer über längere Zeit durch eine kontinuierlich auszuübende Zugkraft entsprechend aufrecht erhaltenen Saugkraft. Die Innenfläche des Hohlkörpers (2) bildet dabei einen Hohlraum (3) aus, der zur Aufnahme eines Penis (1) geeignet ist.

Der luftdichte Abschluss des nur an seinem proximalen Ende (2a) geöffneten Hohlkörpers (2) wird gewährleistet durch eine Hülle (14) - vorzugsweise ein einfaches Standardkondom (5) oder eine handelsübliche Penis-Manschette (6)- welche zwei offene Enden hat. Da die handelsüblichen Kondome natürlich nur proximal eine Öffnung aufweisen, ist es erforderlich, diese an der Kondomspitze aufzuschneiden. Dies ist bei der Verwendung von handelsüblichen Penis-Manschetten (6) dagegen nicht notwendig, da diese ohnehin zwei offene Enden aufweisen. Die Hülle (14) ist mit einer ihrer Öffnungen über einen Teil der Außenfläche (4) des Hohlkörpers (2) und den proximalen Anteil (1a) eines Penis (1) zu stülpen. Sofern es sich bei der Hülle (14) um ein Kondom (5) handelt, muss dieses über einen Teil der Außenfläche (4) des Hohlkörpers (2) und anschließend über den proximalen Anteil (1a) des Penis abgerollt werden. Da der den Penis (1) aufnehmende Hohlkörper (2) zwangsläufig in jedem Fall einen Außendurchmesser hat, der den des Penis (1) deutlich übersteigt, wird durch die hierdurch auf die Außenfläche (4) des Hohlkörpers (2) sehr stark ausgeübte Hüllenkontraktionskraft (des Kondoms (5) bzw. der Penis-Manschette (6)) die Hülle (14) so stark zusammengezogen, dass hierdurch ein absolut luftdichter Abschluss zwischen Hüllen-Innenfläche (8) und Außenfläche (4) des Hohlkörpers (2) erreicht wird, so dass keine Außenluft (9) zwischen Hüllen-Innenfläche (8) und Hohlkörperaußenfläche (4) in den Hohlkörperinnenraum (3) gelangen kann. Die sehr elastische Hülle (14) bewirkt durch ihre Zusammenziehungskraft einen luftdichten Abschluss des Innenraums (3) des Hohlkörpers (2), da die Hülle (14) unmittelbar neben der proximalen Öffnung (2a) des Hohlkörpers (2) auf der Haut des proximalen Anteils (1a) des Penis dichtend anliegt. Durch diese Umhüllung wird verhindert, dass die Außenluft (9) an der Haut des proximalen Anteils (1a) des Penis entlang in den Innenraum (3) des Hohlkörpers (2) eindringen kann. Wenngleich auf diese Weise der erforderliche luftdichte Abschluss des Innenraums (3) des Hohlkörpers (2) bereits erreicht ist, kann das Anpressen des Kondoms (5) an die Haut des proximalen Anteils des Penis (1a) mittels mindestens eines Stücks Latexband (11), Klettband oder Manschette noch unterstützt werden, so dass ein luftdichter Abschluss auch langfristiger sichergestellt werden kann.
Die Zugkraftübertragung erfolgt vorzugsweise durch ein separates Zugband, welches nicht Teil der vorliegenden Erfindung ist und daher nicht dargestellt ist, auf die am Hohlkörper (2) befestigte Zugvorrichtung (7). Da der Zug am Hohlkörper (2) ausgeübt wird - nicht jedoch am Penis direkt oder an einem Kondom- können beliebige Zugvorrichtungselemente wie beispielsweise Haken, Öse, Klemme, Clip, Band, Bindfaden, Faden, Schnur, Sehne, Draht, Kabel, Kordel, Schlaufe, Ring, Schäkel, Zange oder Klettband verwendet werden.
Was die Ausführung des für die Übertragung der Zugkraft auf den Hohlkörper (2) geeigneten Zugkraftübertragungselements (Zugvorrichtung (7)) angeht, so lässt die vorliegende Erfindung erheblichen konstruktiven Freiraum. Hier kommt beispielsweise ein Haken oder eine Öse, eine Klemme oder ein Clip in gleicher Weise in Betracht wie ein Schäkel oder eine Schlaufe. Sind vorerwähnte Zugvorrichtungen bereits am vorerwähnten separaten Zugband montiert, so braucht am Hohlkörper (2) nur ein hierzu passendes Element als Zugvorrichtung (7) befestigt zu sein. Ist am Zugband beispielsweise ein Karabinerhaken montiert, so ist es ausreichend, dass am Hohlkörper (2) als Zugvorrichtung (7) eine Textil-, Kunststoff- oder Metall-Schlaufe befestigt ist, welche in den Karabinerhaken eingehakt wird. Ist am Zugband alternativ ein Clip oder eine Klemme montiert, so sollte am Hohlkörper (2) vorzugsweise eine Zugvorrichtung (7) (beispielsweise ein Metallring) befestigt sein, die vom Clip oder von der Klemme unproblematisch erfasst werden kann. Umgekehrt gilt das gleiche entsprechend: Ist am Hohlkörper (2) beispielsweise ein Karabinerhaken montiert, so ist es ausreichend, dass am separaten Zugband als Zugvorrichtung eine (z.B. Textil- oder Kunststoff-) Schlaufe befestigt ist, welche in den Karabinerhaken eingehakt wird. Ist am Hohlkörper (2) alternativ ein Clip oder eine Klemme montiert, so sollte am separaten Zugband vorzugsweise eine Zugvorrichtung (beispielsweise ein Metallring) befestigt sein, die vom Clip oder von der Klemme unproblematisch erfasst werden kann.
Im Hohlkörperinnenraum (3) der vorliegenden Erfindung wird bei Zug an seiner Zugvorrichtung (7) ein Vakuum und damit ein Unterdruck erzeugt. Der Unterdruck bewirkt eine Saugkraft, die gleichmäßig auf alle an den Hohlkörperinnenraum (3) angrenzenden Flächen verteilt ist. Da der Penis (1) in den Hohlkörperinnenraum (3) eingeführt ist, wird die Saugkraft folglich gleichmäßig auf die Oberfläche des Penis (1) verteilt, welche dem Unterdruck im Hohlkörperinnenraum (3) ausgesetzt ist. Neuere Forschungsergebnisse haben gezeigt, dass bei der Penisdehnung schmerzfreie Mikrofaserrisse resultieren, in die der Körper neues Zellmaterial einbaut. Auf diese Weise ergibt sich eine Verlängerung und Verdickung des Penis.
Die Länge des Hohlkörpers (2) kann beliebig ausgebildet werden, um den individuellen Anforderungen der Anwender gerecht zu werden. Der Hohlkörper (2) kann in seiner Längsrichtung gerade oder gekrümmt sein.

Der zu dehnende Penis liegt während der Dehnungsbehandlung seitwärts gerichtet eng am Körper des Benutzers der vorliegenden Erfindung an. Dies ermöglicht eine Dehnungsbehandlung unter normaler Kleidung, weshalb die Behandlung nicht auf Zeiten, in denen sich der Anwender zuhause aufhält, beschränkt zu werden braucht. Vielmehr kann die Dehnungsbehandlung aufgrund der schmerzlosen, unauffälligen und sehr unproblematischen Anwendung während der täglichen beruflichen Beschäftigung über Stunden erfolgen. Da außerdem die kontinuierliche Dehnung einer vergleichsweise größeren Penisfläche erzielt wird, tritt als Folge hiervon bei einer konsequenten Anwendung der vorliegenden Erfindung eine dauerhafte Vergrößerung des gesamten Penis (nicht nur der Eichel) bereits nach wenigen Monaten ein. Die physische und psychische Wirkung auf den Anwender ist erheblich.

Die vorerwähnte Lösung der gestellten Aufgabe entspricht den Merkmalen von Anspruch 1. Weitere Ausgestaltungen sind entsprechend den Merkmalen des weiteren Anspruchs und der Unteransprüche vorgesehen und unter 'Ausführungsbeispiele' erläutert.

### Zeichnungskennzeichnungsliste

1 = Penis
1a = aus dem Hohlkörper herausragender proximaler Anteil des Penis
1c = Penisspitze (Spitze des Glans penis)
2 = Hohlkörper
2a = proximales offenes Ende des Hohlkörpers zur Aufnahme des Penis
2b = distales geschlossenes Ende des Hohlkörpers
2c = distales offenes Ende des Hohlkörpers ausgebildet zur Befestigung eines Verschlusses (10)
2f = Hohlkörperspitze
2g = Hohlkörpervertiefung
3 = Hohlkörperinnenraum
4 = Hohlkörperaußenfläche
5 = Kondom
5a = Öffnung an der Kondomspitze
6 = Manschette
7= Zugvorrichtung
8 = Hüllen- (Kondom- oder Manschetten-) Innenfläche
9 = Außenluft
10 = Verschluss
11 = Latexband
12 = Hüllenflächenanteil, welcher den zwischen der Außenkante (13) des offenen Endes (2a) des Hohlkörpers und der Oberfläche des proximalen Anteils (1a) des Penis gebildeten Raum dichtend umhüllt
13 = Außenkante der Hohlkörperöffnung
14 = Hülle
15 = Gummiband
16 = Schraubgewinde
17 = Verdickung
18= Unterkante
19=Abbruchteil

### Kurze Beschreibung der Zeichnungen

**Fig. 1** zeigt eine Zugvakuumvorrichtung bestehend aus einem Hohlkörper, einer Hülle dargestellt durch ein Kondom, einer Zugvorrichtung und einem Latexband.
**Fig. 2** zeigt eine Zugvakuumvorrichtung bestehend aus einem Hohlkörper, einer Hülle dargestellt durch eine Manschette und einer Zugvorrichtung.
**Fig. 3** zeigt eine Zugvakuumvorrichtung bestehend aus einem Hohlkörper, einer Hülle, einer an einem Schraub-Verschluss befestigten Zugvorrichtung und einem Latexband.
**Fig. 4** zeigt einen Karabinerhaken als Zugvorrichtung.
**Figs. 5 + 6** zeigen verschiedene Schlaufenausbildungen als Zugvorrichtung.
**Fig. 7** zeigt eine Zugvakuumvorrichtung mit einem sich verjüngenden Hohlkörper, welcher eine Vertiefung aufweist, welche die Befestigung einer Zugvorrichtung ermöglicht.
**Fig. 8** zeigt einen Verschluss mit einer seitlichen Verdickung, welche die Befestigung einer Zugvorrichtung ermöglicht.
**Fig. 9** zeigt einen auf einem Hohlkörper (2) festgeschraubten Verschluss (10), dessen Unterkante die Befestigung einer Zugvorrichtung ermöglicht

### Ausführungsbeispiele

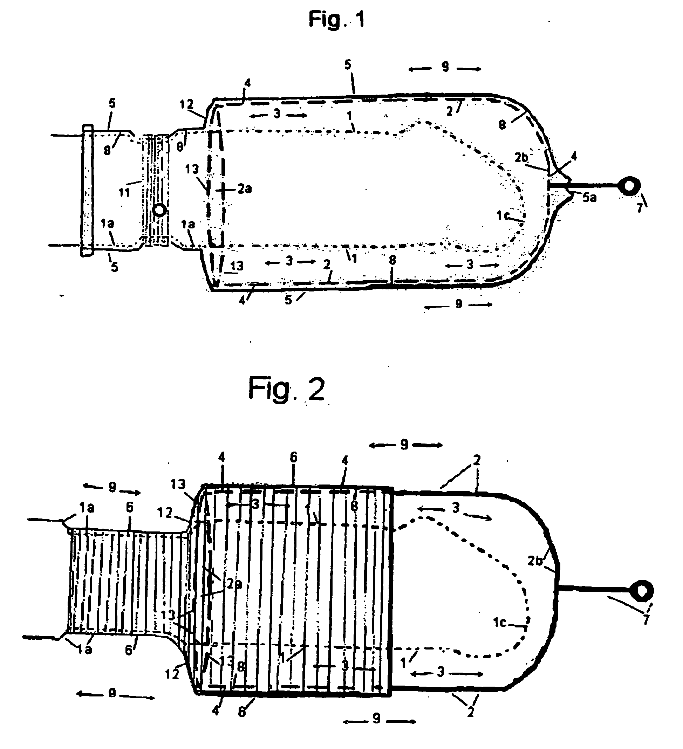

**Fig. 1** zeigt eine Zugvakuumvorrichtung bestehend aus einem Hohlkörper (2) mit einem proximalen offenen Ende (2a) zur Aufnahme eines Penis (1), einer Hülle (Kondom (5)) mit zwei offenen Enden, einer Zugvorrichtung (7) und einem Latexband (11) als zusätzliches Mittel zur hermetischen Abdichtung des proximalen Anteils (1a) des Penis. Alternativ kann über der Hülle -sowohl über dem auf dem Hohlkörper (2), als auch über dem auf dem proximalen Anteil (1a) des Penis liegenden Teil der Hülle (14) - statt mindestens einem Latexband (11) mindestens ein Stück Manschette oder Klettband dichtend befestigt sein. Die erfindungsgemäße Vorrichtung wird folgendermaßen gehandhabt: Sofern es sich bei der Hülle wie in dieser Abbildung um ein Kondom (5) handelt, wird dieses für die erfindungsgemäße Verwendung an seiner Spitze abgeschnitten und die am Hohlkörper (2) befestigte Zugvorrichtung (7) durch die hierdurch geschaffene Öffnung (5a) an der Kondomspitze hindurchgefuhrt Sodann wird das aufgewickelte Kondom mit etwas Kraftaufwand über die distale Hohlkörperaußenfläche (4) gestülpt und über dieser Hohlkörperaußenfläche (4) bis kurz vor dem Erreichen des proximalen offenen Endes (2a) des Hohlkörpers abgerollt. Nach dem Abrollen des Kondoms kann dieses so platzier sein, dass der vordere Teil des Kondoms 5 hinter der öffnung 5a an Kondomspitze auch zumindest einen Teil der Zugvorrichtung 7 umhüllt. Die Kondomspitze kann dabei über das distale Ende des Hohlkörpers (2) hinausragen. Da der den Penis (1) aufnehmende Hohlkörper (2) zwangsläufig in jedem Fall einen Außendurchmesser hat, der den des Penis (1) deutlich übersteigt, wird durch die hierdurch auf die Hohlkörperaußenfläche (4) sehr stark ausgeübte Zusammenziehungskraft des Kondoms (5) dieses so stark zusammengezogen, dass hierdurch ein absolut luftdichter Abschluss zwischen Kondominnenfläche (8) und Hohlkörperaußenfläche (4) erreicht wird. Nun wird der Penis (1) in den Hohlkörper (2) eingeführt. Normalerweise sollte die Einführung des Penis (1) derart erfolgen, dass die Penis-Spitze (1c) am distalen geschlossenen Ende (2b) des Hohlkörpers (2) anstößt oder - wie auf dieser Abbildung - bis kurz vor das geschlossene Ende (2b) geführt wird. Die Funktionsfähigkeit der erfindungsgemäßen Vorrichtung ist allerdings nicht davon abhängig, ob der Penis (1) exakt bis zum distalen geschlossenen Ende (2b) des Hohlkörpers (2) eingeführt wird, da bei der Zugausübung die gewünschte Saugkraft auch bei nicht vollständig eingeführtem Penis (1) erzeugt wird. Sodann wird das Kondom (5) über den Rand des proximalen offenen Endes (2a) des Hohlkörpers hinaus weiter in proximaler Richtung über den aus diesem Ende (2a) herausragenden proximalen Anteil (1a) des Penis abgerollt. Hierbei wird ein Teil des proximalen Anteils (1a) des Penis von der Innenfläche (8) des proximalen Anteils desselben Kondoms (5)aufgrund der Zusammenziehungskraft des Kondoms (5) dichtend luftdicht umhüllt, wobei am proximalen offenen Ende (2a) des Hohlkörpers (2) der nicht vom proximalen Anteil (1a) des Penis ausgefüllte Raum zwischen Außenkante (13) des proximalen offenen Endes (2a) des Hohlkörpers (2) und der Oberfläche des proximalen Anteils (1a) des Penis durch den diesen Raum umhüllenden Flächenanteil (12) des Kondoms (5) luftdicht abgeschlossen ist.

Das Kondom (5) sollte möglichst weit dichtend über den aus dem Hohlkörper (2) herausragenden proximalen Anteil (1a) des Penis abgerollt werden, um einen möglichst vollständigen luftdichten Abschluss des Hohlkörperinnenraums (3) zu erreichen. Das sehr elastische Kondom (5) bewirkt durch seine Zusammenziehungskraft einen luftdichten Abschluss des Hohlkörperinnenraums (3), da das Kondom (5) unmittelbar neben dem proximalen offenen Ende (2a) des Hohlkörpers auf der Haut des proximalen Anteils (1a) des Penis dichtend anliegt und so verhindert, dass die Außenluft (9) an der Haut des proximalen Anteils (1a) des Penis entlang in den Hohlkörperinnenraum (3) eindringen kann.
Die erfindungsgemäße Vorrichtung ist damit bereits voll funktionsfähig. Da ein absolut luftdichter Abschluss auch über längere Zeit die Voraussetzung für ein stundenlanges einwandfreies Funktionieren der vorliegenden Erfindung ist, kann das Anpressen des Kondoms (5) an die Haut des proximalen Anteils (1a) des Penis mittels mindestens eines handelsüblichen Latexbandes (11) oder alternativ durch mindestens ein Stück Manschette oder Klettband noch unterstützt werden, so dass ein absolut luftdichter Abschluss auch langfristiger sichergestellt werden kann. Bei Ausübung einer normalerweise anzuwendenden mäßigen kontinuierlichen Zugkraft ist ein Standard-Kondom (5) vollkommen ausreichend, da ein großer Anteil der ausgeübten Zugkraft in eine Saugkraft umgewandelt wird, so dass nur noch ein kleiner Zugkraftanteil Zug auf das Kondom (5) ausübt, welches im übrigen durch die Saugwirkung an die Penishaut gepresst wird. Nur bei Ausübung größerer und sehr lange andauernder - nur bedingt empfehlenswerter - Zugkräfte sollte entweder eines der ebenfalls handelsüblichen dickwandigeren Kondome bevorzugt werden oder statt einem zwei oder mehr Standard-Kondome verwendet werden, die übereinanderliegen und sich ganz oder teilweise überlappen. Die am Hohlkörper (2) befestigte Zugvorrichtung (7) ist sodann an der entsprechenden Zugvorrichtung eines separaten - nicht zur vorliegenden Erfindung gehörenden und daher nicht dargestellten- Zugbandes zu befestigen. Im Hohlkörperinnenraum (3) der vorliegenden Erfindung wird bei Zug an der Zugvorrichtung (7) des Hohlkörpers (2) ein Vakuum und damit ein Unterdruck erzeugt. Der Unterdruck bewirkt eine Saugkraft, welche die vorgenannten positiven Auswirkungen hat.

**Fig. 2** zeigt eine Zugvakuumvorrichtung bestehend aus einem Hohlkörper (2) mit einem proximalen offenen Ende (2a) zur Aufnahme des Penis (1), einer Hülle (hier dargestellt durch eine handelsübliche Penis-Manschette (6)) mit zwei offenen Enden und einer Zugvorrichtung (7).
Sofern es sich bei der Hülle wie in dieser Abbildung um eine hochelastische Silikon-Manschette (6) mit zwei Öffnungen handelt, ist zunächst der Penis (1) so in den Hohlkörper (2) einzuführen, dass die Penis-Spitze (1c) am distalen geschlossenen Ende (2b) des Hohlkörpers (2) anstößt oder bis kurz vor das geschlossene Ende (2b) geführt wird. Sodann ist die Manschette (6) so über die am Hohlkörper (2) befestigte Zugvorrichtung (7), den Hohlkörper (2) und schließlich den aus dem Hohlkörper (2) herausragenden proximalen Anteil (1a) des Penis zu stülpen, dass die Manschette (6) dichtend einen bis zum proximalen offenen Ende (2a) des Hohlkörpers (2) reichenden Teil der Außenfläche (4) des Hohlkörpers (2) und unmittelbar hinter dem proximalen offenen Ende (2a) des Hohlkörpers (2) dichtend den aus diesem Ende (2a) herausragenden proximalen Anteil des Penis (1a) umhüllt. Der absolut hermetische Abschluss des Hohlkörperinnenraums (3) wird erzielt, wie in Fig. 1 detaillierter erläutert. Statt einer Manschette mit großer länglicher Ausdehnung können auch zwei oder mehr übereinanderliegende kürzere Manschetten, die sich teilweise überlappen, Verwendung finden. Die erfindungsgemäße Vorrichtung ist damit bereits voll funktionsfähig. Die am Hohlkörper (2) befestigte Zugvorrichtung (7) ist sodann vorzugsweise an einem separaten - nicht zur vorliegenden Erfindung gehörenden -Zugband zu befestigen. Durch den im Hohlkörperinnenraum (3) durch Zug an der Zugvorrichtung (7) erzeugten Unterdruck werden Saugkräfte auf den Hohlkörperinnenraum (3) und damit auf den hierin eingeführten Penis (1) ausgeübt. Um das Zusammenquetschen des Hohlkörpers (2) bei größerem Unterdruck zu vermeiden, sollte daher normalerweise starres Material für den Hohlkörper (2) Verwendung finden. Allerdings ist es auch von der Form des Hohlkörpers (2) abhängig, ob derselbe beim Ausüben einer größeren Zugkraft verformt oder gar zusammengequetscht wird, da der Unterdruck gleichmäßig an allen Punkten des Hohlkörperinnenraums (3) ansetzt. Danach können auch elastische Materialien Verwendung finden. Schließlich kann es sogar erwünscht sein, dass der Hohlkörper (2) sich dann verformt, wenn eine bestimmte Zugkraft überschritten wird. Um nämlich eine solche Überschreitung zu verhindern, kann elastisches Material erwünscht sein, das durch seine Verformung ab einer bestimmten auf die Zugvorrichtung (7) ausgeübten Zugkraft dem Anwender das Signal gibt, die Zugkraft zu vermindern. Der Hohlkörper kann daher aus einem beliebigen Material gefertigt sein, das sowohl starr als auch elastisch sein kann. Beispielsweise können Kunststoff, Glas, Gummi, Metall, Keramik, Holz oder Verbundstoff für die Herstellung des Hohlkörpers verwendet werden. Auch die Form des Hohlkörpers (2) kann beliebig gestaltet werden. Beispielsweise kann vorgesehen sein, dass sich der Hohlkörper in Richtung auf sein distales geschlossenes Ende (2b) verdickt, verjüngt oder seine Außenmaße sich nicht verändern, so dass sich sein Durchmesser in Richtung auf sein distales Ende (2b) vergrößert, verkleinert oder nicht verändert. Der Hohlkörper (2) kann eine beliebige Behälterform - beispielsweise eine Dosen-, Flaschen-, Zylinder-, Röhren-, Becher- oder Tubenform - aufweisen.

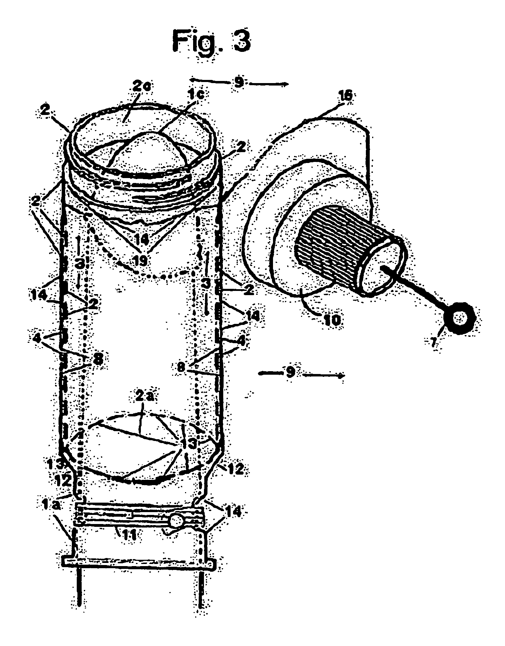

**Fig. 3** zeigt eine Zugvakuumvorrichtung bestehend aus einem Hohlkörper (2) mit einem proximalen offenen Ende (2a) zur Aufnahme des Penis (1) und einem distalen offenen Ende (2c) ausgebildet für das Verschließen dieses Endes (2c) durch einen Schraub-Verschluss (10), einer Hülle (14) mit zwei offenen Enden, auf deren über dem proximalen Anteil (1a) des Penis gelegenen proximalen Hüllenanteil zusätzlich mindestens ein handelsübliches Latexband (11) (oder alternativ mindestens ein Stück Manschette oder Klettband) dichtend festgezurrt werden kann, einen mit Hilfe eines Gummibandes (15) mit dem Hohlkörper (2) verbundenen Schraub-Verschluss (10) und einer am Schraub- Verschluss (10) befestigten Zugvorrichtung (7).
Die hier dargestellte Hülle (14) kann sowohl ein Kondom als auch alternativ eine (handelsübliche Penis-Silikon-) Manschette sein. Ein Hohlkörper (2) mit einem Verschluss (10) hat mehrere Vorzüge: 1) Problemloses, hygienisches Wasser lassen wird ermöglicht durch einfaches Öffnen des Verschlusses (10). Wie aus dieser Fig. 3 ersichtlich, kann uriniert werden, ohne den Hohlkörper (2) oder den Verschluss (10) zu benetzen, zumal der Hohlkörper (2) beim Urinieren etwas in proximaler Richtung bewegt werden kann. 2) Sofern die Zugvakuumvorrichtung vom Glied entfernt werden soll, ist dies - nach Abnahme des Latexbandes (11) - leicht nach Öffnen des Verschlusses (10) möglich, da so beim Abstreifen des Hohlkörpers (2) kein Unterdruck erzeugt wird, welcher die Hülle (14) am Penis festsaugen würde. Die Verwendung eines Hilfsmittels für das Abstreifen ist hier daher nicht erforderlich. 3) Bei Abnahme des Verschlusses (10) kann der Anwender zumindest die Penisspitze (1c) und die Vorhaut auf eventuell vorhandene Hautveränderungen inspizieren und bei ihrem Vorhandensein eine Pause bei der Benutzung der vorliegenden Erfindung einlegen. 4) Die meisten Verschlüsse sind gut geeignet, um an ihnen oder ihren Abbruchteilen (19) verschiedenartig ausgebildete Zugvorrichtungen zu befestigen. Der Verschluss kann beliebig ausgebildet sein. Beispielsweise kann der Verschluss ein Schraub-, Kipp-, Dreh-, Bajonett-, Steck-, Klemm-, Stopfen-, Druck-, Schnapp-, Schwenk-, Clip-, Klett- oder Rast-Verschluss sein.

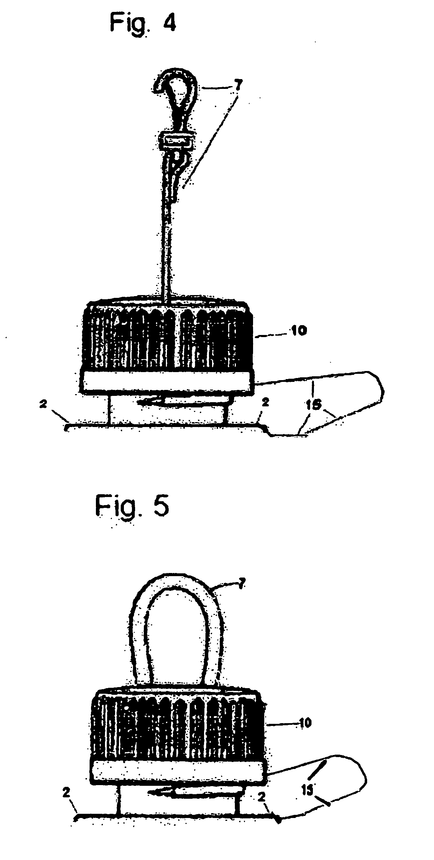

**Fig. 4** zeigt einen Karabinerhaken als Zugvorrichtung.
Hier ist die Zugvorrichtung (7) ein mittels eines Drahtes am Deckel eines Schraub-Verschlusses (10) befestigter Karabinerhaken, welcher seinerseits mittels eines Gummibandes (15) mit dem Hohlkörper (2) verbunden ist, sofern er vom Hohlkörper (2) abgeschraubt wird. Die Zugvorrichtung (7) kann alternativ mindestens ein Stück Haken, Öse, Klemme, Clip, Bindfaden, Faden, Faser, Band, Schnur, Sehne, Draht, Kabel, Kordel, Schlaufe, Ring, Schäkel, Zange oder Klettband aufweisen.

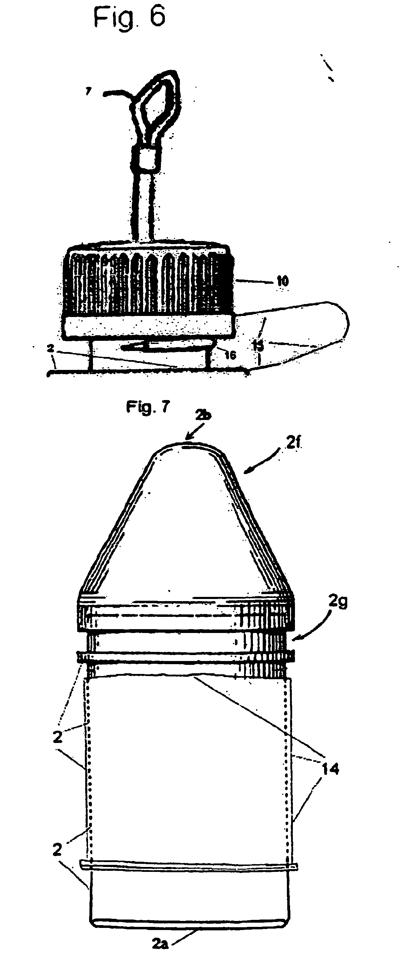

**Fig. 5** zeigt eine mit ihren Enden direkt am Deckel eines Schraub-Verschlusses (10) befestigte Zugvorrichtung (7) (hier eine Schlaufe), während auf **Fig. 6** eine Zugvorrichtung (7) dargestellt ist, deren beide Schlaufenenden an einer Kordel befestigt sind, welche ihrerseits am Deckel eines Schraub-Verschlusses (10) fixiert ist. Der Schraub- Verschluss (10) ist seinerseits mittels eines Gummibandes (15) mit dem Hohlkörper (2) verbunden. Die in Figs. 4-6 beispielhaft dargestellten Zugvorrichtungen (7) können statt an einem beliebig gearteten Verschluss (10) auch am Hohlkörper (2) durch beliebige Befestigungsmittel befestigt werden, und zwar vorzugsweise an der Außenfläche des distalen geschlossenen Endes (2b) des Hohlkörpers (2). Alternativ ist die Befestigung der Zugvorrichtung (7) auch an der seitlichen Außenfläche, der Außenkante (13) des offenen Endes (2a) oder der Innenfläche des Hohlkörpers (2) möglich.
Beispielsweise kann auch an dem für einen Schraub-Verschluss (10) am Hohlkörper (2) vorgesehenen Schraubgewinde (16) das Element einer Zugvorrichtung (z.B. eine Schlaufe) durch eine beliebiges Befestigungsmittel befestigt werden bzw. die Befestigung kann auch nur dadurch herbeigeführt werden, dass der Verschluss (10) beim Verschließen das Element einer Zugvorrichtung einklemmt. Die beim Verschließen hervorgerufene Presskraft ermöglicht dabei die Befestigung mindestens eines Elementes einer Zugvorrichtung, die entweder Teil der vorliegenden Erfindung oder eines separaten Zugbandes sein kann, am Schraubverschluss (10) oder einem beliebigen anderen Verschluss.

**Fig. 7** zeigt eine Zugvakuumvorrichtung mit einem sich in Richtung auf sein distales geschlossenes Ende (2b) verjüngenden Hohlkörper (2), wobei der Hohlkörper (2) eine Vertiefung (2g) aufweist.

Der sich in Richtung auf seine Spitze (2f) verjüngende Hohlkörper (2) weist hinter seiner Spitze (2f), die der Form einer Penisspitze ähnelt, eine Vertiefung (2g) auf. Diese kann von einer Zugvorrichtung des separaten - nicht zur vorliegenden Erfindung gehörenden - Zugbandes erfasst werden. Diese Zugvorrichtung kann beispielsweise als zangenähnliches Element oder als sich in dieser Vertiefung einrastendes Element ausgebildet sein. Über den Hohlkörper (2) ist eine Hülle (14) (hier ein Kondom) gestülpt. Das Kondom ist bis kurz vor das proximale offene Ende (2a) abgerollt. Der Hohlkörper (2) ist damit bereit für die Einführung eines Penis in das offene Ende (2a).

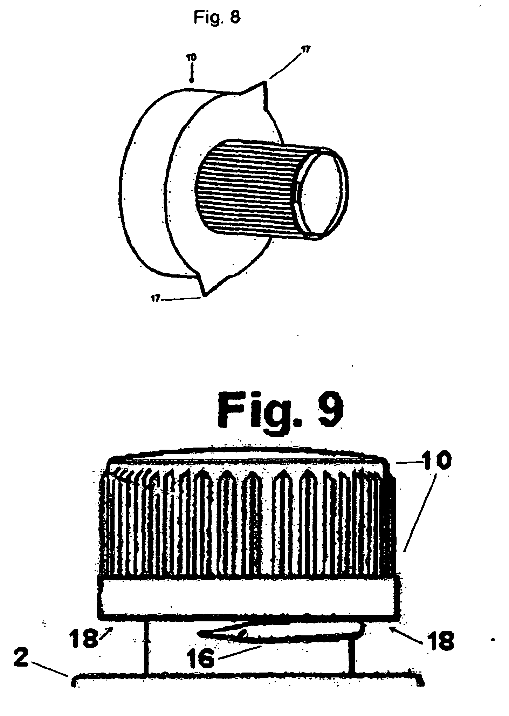

**Fig. 8** zeigt einen Verschluss (10) mit einer seitlichen Verdickung (17), welche die Befestigung einer Zugvorrichtung ermöglicht.

Der Hohlkörper (2) oder der Verschluss (10) können in einer weiteren Ausführungsform so gestaltet sein, dass auf sie Zug ausgeübt werden kann, ohne dass eine Erfordernis für das Vorhandensein einer an der vorliegenden Erfindung montierten Zugvorrichtung (7) besteht. Die Zugausübung kann ermöglicht werden durch das Vorhandensein von Ausbildungen an den Wänden des Hohlkörpers (2) oder des Verschlusses (10) oder an einem Abbruchteil ((19) in Fig. 3)) des Hohlkörpers (2) oder des Verschlusses (10), die beispielsweise von einer am separaten - nicht zur vorliegenden Erfindung gehörenden - Zugband befestigten zangenartigen Vorrichtung, einem großen Clip, einer Schnapp- oder Einrastvorrichtung erfasst werden können. Solche speziellen Ausbildungen können beispielsweise sein: Mindestens eine Verdickung, Vertiefung, Öffnung, Steg, Gewinde oder Kante. Eine die Kante betreffende Ausbildungsalternative ist in Fig. 9 genannt.

Bekanntlich kann an einem Verschluss oder Hohlkörper, ein - vorzugsweise ringförmiges - Abbruchteil (19) mit einer Sollbruchstelle versehen sein. Beim Öffnen des Verschlusses löst sich dieses Teil normalerweise vollständig oder auch nur teilweise vom Verschluss bzw. Hohlkörper. Dieses Abbruchteil (19) verbleibt am Verschluss (10) oder Hohlkörper (2) und hat nur die Signalwirkung, dass der Inhalt des Hohlkörpers (2) angebrochen wurde, aber normalerweise keine weitere Funktion. Jetzt kann dieses Abbruchteil (19) aber die Funktion der Befestigung einer Zugvorrichtung (7) der vorliegenden Erfindung oder einer Zugvorrichtung des separaten - nicht zur vorliegenden Erfindung gehörenden - Zugbandes bekommen. Beispielsweise indem durch das ringförmige Abbruchteil mindestens ein Loch getrieben wird, durch welches beispielsweise ein zu der Zugvorrichtung (7) der vorliegenden Erfindung oder des separaten - nicht zur vorliegenden Erfindung gehörenden - Zugbandes gehöriger Draht geführt wird. Der durch das Loch geführte und hieran befestigte Draht gibt so dem Abbruchteil (19) eine für die vorliegende Erfindung sinnvolle Funktion.

**Fig. 9** zeigt einen auf einem Hohlkörper (2) festgeschraubten Verschluss (10), dessen Unterkante die Befestigung einer Zugvorrichtung ermöglicht.
Auf die Unterkante eines Schraub-Verschlusses (10) kann nach dem Festschrauben des Verschlusses (10) auf dem Hohlkörper (2) Zug durch eine der in Fig. 8 erwähnten Vorrichtungen ausgeübt werden, sofern die Unterkante (18) so ausgebildet ist, dass sie genügend weit von dem mit einem Schraubgewinde (16) versehenen Hohlkörper (2) absteht, um das Erfassen durch die in Fig. 8 erwähnten Vorrichtungen zu ermöglichen. Alternativ kann durch die beim Festschrauben des Verschlusses (10) erzeugte Verschlusspresskraft die Befestigung einer Zugvorrichtung (7) dieser Erfindung oder eines separaten Zugbandes am Hohlkörper (2), Verschluss (10), Abbruchteil (19) oder an diesen angebrachten Ausbildungen erfolgen.

## Patentansprüche

1. Zugvakuumvorrichtung zur Penisextension, **dadurch gekennzeichnet, dass** diese einen Hohlkörper (2) mit einem offenen Ende (2a) zur Aufnahme eines Penis (1), eine Hülle (14) mit zwei offenen Enden und eine Vorrichtung zur Zugausübung auf den Hohlkörper aufweist, wobei ein Teil der Hülle (14) über der Hohlkörperaußenfläche (4) und ein anderer Teil derselben Hülle (14) über der Außenfläche des aus dem Hohlkörper (2) herausragenden proximalen Anteils (1a) des Penis angeordnet ist und Hohlkörper (2) und Penis (1) dabei dichtend umhüllend miteinander verbindet, wobei am proximalen offenen Ende (2a) des Hohlkörpers (2) der nicht von dem aus dem Hohlkörper (2) herausragenden proximalen Anteil (1a) des Penis ausgefüllte Raum zwischen Außenkante (13) des proximalen offenen Endes (2a) des Hohlkörpers (2) und der Oberfläche des proximalen Anteils (1a) des Penis durch den diesen Raum umhüllenden Flächenanteil (12) der Hülle (14) luftdicht abgeschlossen ist.

2. Zugvakuumvorrichtung zur Penisextension, **dadurch gekennzeichnet, dass** diese einen Hohlkörper (2) mit einem proximalen offenen Ende (2a) zur Aufnahme eines Penis (1) und einem für die Befestigung eines Verschlusses (10) ausgebildeten offenen distalen Ende (2c), eine Hülle (14) mit zwei offenen Enden, einen Verschluss (10) und eine Vorrichtung zur Zugausübung auf den Hohlkörper (2), auf den Verschluss (10) oder auf ein Abbruchteil (19) des Hohlkörpers (2) oder Verschlusses (10) aufweist, wobei ein Teil der Hülle (14) über der Hohlkörperaußenfläche (4) und ein anderer Teil derselben Hülle (14) über der Außenfläche des aus dem Hohlkörper (2) herausragenden proximalen Anteils (1a) des Penis angeordnet ist und Hohlkörper (2) und Penis (1) dabei dichtend umhüllend miteinander verbindet, wobei am proximalen offenen Ende (2a) des Hohlkörpers (2) der nicht von dem aus dem Hohlkörper (2) herausragenden proximalen Anteil (1a) des Penis ausgefüllte Raum zwischen Außenkante (13) des proximalen offenen Endes (2a) des Hohlkörpers (2) und der Oberfläche des proximalen Anteils (1a) des Penis durch den diesen Raum umhüllenden Flächenanteil (12) der Hülle (14) luftdicht abgeschlossen ist.

3. Zugvakuumvorrichtung nach einem der Ansprüche 1 - 2, **dadurch gekennzeichnet, dass** die Vorrichtung zur Zugausübung eine am Hohlkörper (2), dem Verschluss (10) oder einem Abbruchteil (19) des Hohlkörpers (2) oder Verschlusses (10) durch Befestigungsmittel oder nur durch eine Verschlusspresskraft befestigte Zugvorrichtung (7) oder mindestens eine am Hohlkörper (2), dem Verschluss (10) oder einem Abbruchteil (19) des Hohlkörpers (2) oder Verschlusses (10) angebrachte Ausbildung ist, welche die Befestigung einer Zugvorrichtung ermöglicht.

4. Zugvakuumvorrichtung nach einem der Ansprüche 1-3, **dadurch gekennzeichnet, dass** die Ausbildung mindestens eine Verdickung, Vertiefung, Öffnung, Steg, Gewinde oder abstehende Kante aufweist.

5. Zugvakuumvorrichtung nach einem der Ansprüche 1 - 3, **dadurch gekennzeichnet, dass** die Zugvorrichtung (7) an der Außenfläche des distalen geschlossenen Endes (2b), der seitlichen Außenfläche, der Außenkante (13) des offenen Endes (2a) oder der Innenfläche des Hohlkörpers (2) befestigt ist.

6. Zugvakuumvorrichtung nach einem der Ansprüche 2-5, **dadurch gekennzeichnet, dass** der Verschluss (10) ein Schraub-, Kipp-, Dreh-, Bajonett-, Steck-, Klemm-, Stopfen-, Druck-, Schnapp-, Schwenk-, Clip-, Klett- oder Rast-Verschluss ist.

7. Zugvakuumvorrichtung nach einem der Ansprüche 1-3 und 5+6, **dadurch gekennzeichnet, dass** die Zugvorrichtung (7) mindestens ein Stück Haken, Öse, Klemme, Clip, Band, Bindfaden, Faden, Schnur, Sehne, Draht, Kabel, Kordel, Schlaufe, Ring, Schäkel, Zange oder Klettband aufweist.

8. Zugvakuumvorrichtung nach einem der Ansprüche 1 -7, **dadurch gekennzeichnet, dass** der Hohlkörper (2) aus Kunststoff-, Glas-, Gummi-, Metall-, Keramik-, Holz- oder Verbundstoffmaterial gefertigt ist, wobei das Material elastisch oder starr ist, und eine Dosen-, Flaschen-, Zylinder-, Röhren-, Becher- oder Tubenform aufweist, wobei sein Längsschnitt gerade oder gekrümmt und sein Querschnitt rund, dreieckig, rechteckig oder vieleckig ist, wobei sich sein Durchmesser in Richtung auf sein distales Ende (2b) vergrößert, verkleinert oder nicht verändert.

9. Zugvakuumvorrichtung nach einem der Ansprüche 1-8, **dadurch gekennzeichnet, dass** der Hohlkörper (2) Perforationen oder Sollbruchstellen derart aufweist, dass die Länge des Hohlkörpers (2) durch Abreißen von Teilen des Hohlkörpers (2) an den Perforationen oder Sollbruchstellen auf eine gewünschte Länge kürzbar ist.

10. Zugvakuumvorrichtung nach einem der Ansprüche 1-9, **dadurch gekennzeichnet, dass** die Innenfläche des Hohlkörpers (2) einen Hohlraum (3) ausbildet, der zur Aufnahme eines Penis (1) geeignet ist.

11. Zugvakuumvorrichtung nach einem der Ansprüche 1-10 , **dadurch gekennzeichnet, dass** die Hülle (14) ein Kondom (5) oder eine Manschette (6) ist oder aus mindestens zwei übereinanderliegenden Kondomen oder Manschetten gebildet wird.

12. Zugvakuumvorrichtung nach einem der Ansprüche 1-11, **dadurch gekennzeichnet, dass** auf der Hülle (14) mindestens ein Stück Latexband (11), Manschette oder Klettband dichtend aufliegt.

13. Zugvakuumvorrichtung nach einem der Ansprüche 1-12, **dadurch gekennzeichnet, dass** die Hülle (14) auch zumindest einen Teil der Zugvorrichtung (7) umhüllt.

14. Zugvakuumvorrichtung nach einem der Ansprüche 1-13, **dadurch gekennzeichnet, dass** die Hülle (14) über das distale Ende des Hohlkörpers (2) hinausragt.

## Claims

1. Device for extending a penis by applying vacuum generated by pulling, **characterized in that** same has a hollow body (2) with an open end (2a) for the introduction of a penis (1), a jacket (14) with two open ends and a means (7) for exerting a tractive force on the hollow body (2), whereby a part of the jacket (14) is positioned over the outer surface (4) of the hollow body (2) and another part of the same jacket (4) over the outer surface of the proximal part (1a) of the penis protruding from the hollow body (2), thereby connecting hollow body (2) and penis (1) with each other and tightly wrapping same, whereby the space between the outer edge (13) of the proximal open end (2a) of the hollow body (2) and the surface of the proximal part (1a) of the penis, which is not filled by the proximal part (1a) of the penis protruding from the hollow body (2) is hermetically sealed by the part (12) of the jacket which covers this space.

2. Device for extending a penis by applying vacuum generated by pulling, **characterized in that** same has a hollow body (2) with a proximal open end (2a) for the introduction of a penis (1) and an open distal end (2c) formed for the fastening of a closure (10), a jacket (14) with two open ends, a closure (10) and means (7) for exerting a tractive force on the hollow body (2), on the closure (10) or on a break-off part (19) of the hollow body (2) or the closure (10), whereby a part of the jacket (14) is positioned over the outer surface (4) of the hollow body (2) and another part of the same jacket (14) is positioned over the outer surface of the proximal part (1a) of the penis protruding from the hollow body (2), thereby connecting hollow body (2) and penis (1) with each other and tightly wrapping same, whereby the space between the outer edge (13) of the proximal open end (2a) of the hollow body (2) and the surface of the proximal part (1a) of the penis, which is not filled by the proximal part (1a) of the penis protruding from the hollow body (2) is hermetically sealed by the part (12) of the jacket which covers this space.

3. Device according to one of the claims 1-2, **characterized in that** the means for exerting a tractive force is a pulling device (7) attached by fastening means or only by means of the pressing power of the closure (10) to the hollow body (2), the closure (10) or to a break-off part (19) of the hollow body (2) or of the closure (10), or at least one extension mounted to the hollow body (2), to the closure (10) or to the break-off part (19) of the hollow body (2) or of the closure (10), which enables the engaging of pulling means.

4. Device according to one of the claims 1-3, **characterized in that** the extension has at least one thickening, deepening, opening, plate, thread or prominent edge.

5. Device according to one of the claims 1-3, **characterized in that** the pulling device (7) is fastened to the outer surface of the distal closed end (2b), the lateral outer surface, the outer edge (13) of the open end (2a) or the inner surface of the hollow body (2).

6. Device according to one of the claims 2-5, **characterized in that** the closure (10) is a screw closure, tipping closure, turning closure, bayonet closure, prong closure, clamp closure, plug closure, pressure closure, snap closure, swinging closure, clip closure, Velcro closure or click-lock closure.

7. Device according to one of the claims 1-3 and 5-6, **characterized in that** the pulling device (7) has at least one piece of hook, eye, clamp, clip, band, twine, thread, string, tendon, wire, cable, cord, loop, ring, shackle, tong or Velcro band.

8. Device according to one of the claims 1-7, **characterized in that** the hollow body (2) is manufactured from plastic, glass, rubber, metal, ceramic, wood or compound material, whereby the material is elastic or rigid and has the form of a box, bottle, cylinder, tube, cup or collapsible tube, whereby his longitudinal section is straight or bent and his cross section is round, triangular, rectangular or polygonal, whereby his diameter increases, decreases or remains unchanged in the direction of his distal end (2b).

9. Device according to one of the claims 1-8, **characterized in that** the hollow body (2) has perforations or breakpoint areas allowing that the length of the hollow body (2) is shortened to the desired length by tearing off parts of the hollow body (2).

10. Device according to one of the claims 1-9, **characterized in that** the inner surface of the hollow body (2) forms a hollow space suitable for the introduction of a penis (1).

11. Device according to one of the claims 1-10, **characterized in that** the jacket (14) is a condom (5) or a sleeve (6), or is formed by at least two condoms or sleeves superimposed one upon another.

12. Device according to one of the claims 1-11, **characterized in that** the jacket (14) is tightly covered by at least one piece of Latex band (11), sleeve or Velcro band.

13. Device according to one of the claims 1-12, **characterized in that** the jacket (14) also covers at least a part of the pulling device (7).

14. Device according to one of the claims 1-13, **characterized in that** the jacket (14) projects beyond the distal end of the hollow body (2).

## Revendications

1. Mécanisme de vacuum à traction pour l'extension du pénis, **caractérisé par** la présence d'un corps creux (2) avec un bout ouvert (2a) qui peut incorporer un pénis (1), d'une gaine (14) avec deux bouts ouverts et un mécanisme qui fait traction sur le corps creux (2), dont une partie de la gaine (14) se trouve sur la surface extérieure (4) du corps creux (2) et l'autre part de la même gaine (14) se trouve au-dessus de la surface de la part proximale (1a) du pénis qui émerge du corps creux (2), ce qui joint et gaine hermétiquement le pénis (1) et le corps creux (2), dont l'espace n'est pas rempli par la part proximale (1a) du pénis qui émerge du corps creux (2) à son bout proximale ouvert (2a), qui se trouve entre le bord extérieur (13) du bout ouvert proximale (2a) du corps creux (2) et la surface de la part proximale (1a) du pénis, et qui est fermée hermétiquement par la part du surface (12) de la gaine (14).

2. Mécanisme de vacuum à traction pour l'extension du pénis, **caractérisé par** l'existence d'un corps creux (2) avec un bout ouvert proximale (2a) pour incorporer un pénis (1) et un bout ouvert distal (2c) formé pour y fixer une fermeture (10), une gaine (14) avec deux bouts ouverts, une fermeture (10) et une construction pour faire traction au corps creux (2), à la fermeture (10) ou à une partie abrupte (19) du corps creux (2) ou de la fermeture (10), dont une partie de la gaine (14) se trouve sur la face extérieure (4) du corps creux (2) et une autre partie de la même gaine (14) se trouve sur la face extérieure de la part proximale (1a) du pénis qui émerge du corps creux (2) et qui joint et gaine hermétiquement le corps creux (2) et le pénis (1), dont l'espace n'est pas rempli par la part proximale (1a) du pénis qui émerge du corps creux (2) à son bout proximale ouvert (2a), qui se trouve entre le bord extérieur (13) du bout ouvert proximale (2a) du corps creux (2) et la surface de la part proximale (1a) du pénis, et qui est fermée hermétiquement par la part du surface (12) de la gaine (14).

3. Mécanisme de vacuum à traction selon un des droits de patente 1-2, dont le mécanisme pour exercer de la traction est **caractérisé par** un dispositif de traction appliqué au corps creux (2), à la fermeture (10) ou une partie abrupte (19) du corps creux (2) ou de la fermeture (10) par des moyen de fixage ou seulement par une force de fermeture-pression ou qui est au moins une extension appliquée au corps creux (2), à la fermeture (10) ou une partie abrupte (19) du corps creux (2) ou de la fermeture (10), qui rend possible de fixer le mécanisme de traction.

4. Mécanisme de vacuum à traction selon un des droits de patente 1 -3, **caractérisé par** l' attribut que l'extension a au moins une callosité, concavité, ouverture, traverse, une filetage ou un bord éloigné.

5. Mécanisme de vacuum à traction selon un des droits de patente 1 -3, **caractérisé par** l'attribut que le mécanisme de traction (7) est fixé à l'extérieur du bout fermé distal (2b), de la surface latérale, le bord extérieur (13) du bout ouvert (2a) ou à 1 surface intérieure du corps creux (2).

6. Mécanisme de vacuum à traction selon un des droits de patente 2-5, **caractérisé par** l'attribut que la fermeture (10) est une fermeture à vis, à bouger, à tourner, à baïonnette, à enfoncer, à bornes, à pousser, à presser, à cliquet, à pivoter, à clip, à scratch ou à encliqueter.

7. Mécanisme de vacuum à traction selon un des droits de patente 1-3 et 5-6, **caractérisé par** l'attribut que le mécanisme de traction (7) a au moins une pièce de crochet, boucle, borne, clip, bande, ficelle, fil, attache, tendon, fil de fer, câble, cordelette, dragonne, anneau, manille, blochet ou ruban-crochets.

8. Mécanisme de vacuum à traction selon un des droits de patente 1 -7, **caractérisé par** l'attribut que le corps creux (2) est produit d'un matériel plastique, de verre, de caoutchouc, de métal, de céramique, de bois ou matériel composite, dont le matériel et élastique ou rigide, et a une forme de boîte, bouteille, cylindre, tube, gobelet ou tuyau, dont sa section longitudinale est linéale ou incurvé et dont sa coupe transversale est rond, triangulaire, rectangulaire ou polygonale, dont son diamètre s'étend, se diminue ou ne change pas en direction de son bout distal (2b).

9. Mécanisme de vacuum à traction selon un des droits de patente 1-8, **caractérisé par** l'attribut que le corps creux (2) a des perforations ou des points de rupture théorétique d'une manière que la longueur du corps creux (2) peut être abrégé par tirer des parties du corps creux (2) aux perforations ou points de rupture théorétique à la longueur souhaitée.

10. Mécanisme de vacuum à traction selon un des droits de patente 1-9, **caractérisé par** l'attribut que la surface intérieure du corps creux (2) forme un vide (3), qui est apte à contenir un pénis (1).

11. Mécanisme de vacuum à traction selon un des droits de patente 1-10 , **caractérisé par** l'attribut que la gaine (14) est un préservatif (5) ou une manchette (6) ou est formé au moins de deux préservatifs ou de manchettes chevauché(e)s.

12. Mécanisme de vacuum à traction selon un des droits de patente 1-11, **caractérisé par** l'attribut qu'il est appliqué sur la gaine (14) au moins une pièce de bande latex (11), de manchette ou de ruban-crochets.

13. Mécanisme de vacuum à traction selon un des droits de patente 1-12, **caractérisé par** l'attribut que la gaine (14) enveloppe aussi au moins une part du mécanisme de traction (7).

14. Mécanisme de vacuum à traction selon un des droits de patente 1-13, **caractérisé par** l'attribut que la gaine (14) s'étend au-delà du bout distal du corps creux (2).
